(19) Europäisches Patentamt
European Patent Office
Office européen des brevets

(11) Veröffentlichungsnummer: **0 278 900 A2**

(12) # EUROPÄISCHE PATENTANMELDUNG

(21) Anmeldenummer: **88810038.5**

(51) Int. Cl.4: **C 08 G 59/06**

(22) Anmeldetag: **25.01.88**

(30) Priorität: **30.01.87 CH 352/87**

(43) Veröffentlichungstag der Anmeldung: **17.08.88 Patentblatt 88/33**

(84) Benannte Vertragsstaaten: **CH DE ES FR GB IT LI NL**

(71) Anmelder: **CIBA-GEIGY AG**
**Klybeckstrasse 141**
**CH-4002 Basel (CH)**

(72) Erfinder: **Roth, Martin, Dr.**
**Oberdorf**
**CH-1711 Giffers (CH)**

**Monnier, Charles E., Dr.**
**Ch. du Verger 16**
**CH-1752 Villars-sur-Glâne (CH)**

(54) **Vorverlängerte Epoxidharze auf Basis von Cyclohex-1-ylmethylendiphenolderivaten oder Bicyclo[2.2.1]hept-1-ylmethylen-diphenolderivaten.**

(57) Die Erfindung betrifft Verbindungen der Formeln I oder II

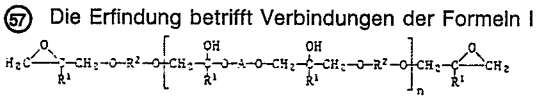 (I),

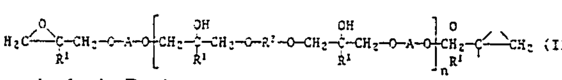 (II),

worin A ein Rest

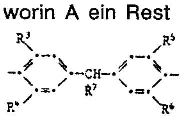

ist, $R^1$ Wasserstoff oder Methyl bedeutet,
$R^2$ der Rest eines aliphatischen, cycloaliphatischen, aromatischen oder araliphatischen Diols nach dem Entfernen beider Hydroxylgruppen ist,
$R^3$, $R^4$, $R^5$ und $R^6$ unabhängig voneinander Wasserstoff, $C_1$-$C_6$-Alkyl, Chlor oder Brom bedeuten,
$R^7$ ein Rest der Formeln III, IV, V oder VI ist

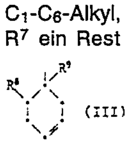 (III), 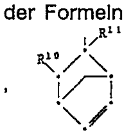 (IV), 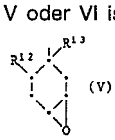 (V), 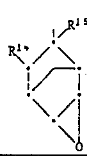 (VI),

$R^8$, $R^{10}$, $R^{12}$ und $R^{14}$ Wasserstoff, $C_1$-$C_6$-Alkyl oder Phenyl sind,
$R^9$, $R^{11}$, $R^{13}$ und $R^{15}$ Wasserstoff oder $C_1$-$C_6$-Alkyl bedeuten und der mittlere Wert von n (Zahlenmittel) eine Zahl von 1 bis 20 ist,
wobei die Reste $R^1$ bis $R^{15}$ innerhalb eines gegebenen Moleküls im Rahmen der gegebenen Definitionen unterschiedliche Bedeutungen annehmen können.

Diese Verbindungen oder auch die epoxidierten Zwischenprodukte der Formel X

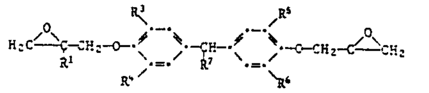 (X),

worin $R^1$ bis $R^3$ die oben definierte Bedeutung besitzen, und $R^7$ ein Rest der Formeln IV oder VI ist lassen sich zu gehärteten Produkten mit hoher Glasumwandlungstemperatur und geringer Verfärbungstendenz verarbeiten.

EP 0 278 900 A2

**Beschreibung**

Vorverlängerte Epoxidharze auf Basis von Cyclohex-1-ylmethylendiphenolderivaten oder Bicyclo[2.2.1]hept-1-ylmethylendiphenolderivaten

Die vorliegende Erfindung betrifft vorverlängerte Epoxidharze, die sich von speziellen Diphenolen bzw. von Diglycidylethern auf Basis dieser Phenole ableiten, ein Verfahren zur Herstellung der speziellen Diephenole, die neuen Glycidyletherzwischenprodukte und härtbare Mischungen enthaltend die vorverlängerten Derivate und/oder die Zwischenprodukte in Kombination mit Härtern.

Cyclohex-1-ylmethylendiphenole sind aus dem GB-Patent 1,024,012 bekannt. Bicyclo[2.2.1]hept-1-ylmethylendiphenole werden im GB-Patent 1,024,013 beschrieben. Diese Verbindungen werden beispielsweise als Monomerkomponenten bei der Polycarbonatherstellung eingesetzt. Solche Umsetzungen werden im GB-Patent 1,024,011 beschrieben. Die Herstellung von Glycidylethern von Hydroxyaryl-3,4-epoxycyclohexylmethanen wird von B.M. Tkatschuk et al. beschrieben (vgl. Ref. Zh. Khim., 1985, Abstr. Nr. 19S419). Die hort hergestellten Verbindungen sind niedermolekular und besitzen demzufolge eine relativ niedrige Viskosität. Für gewisse Anwendungen, z.B. Laminate, sind höher aufgebaute Oligomere von Vorteil.

Es wurde jetzt eine Klasse von vorverlängerten Epoxidharzen gefunden, die sich gegenüber herkömmlichen vorverlängerten Epoxidharzen auf Bisphenol-A Basis durch vorteilhafte Eigenschaften auszeichnet. So besitzen die gehärteten Produkte aus diesen neuen Harzen in der Regel hohe Glasübergangstemperaturen; ausserdem erhält man bei der Härtung mit Imidazol-Beschleunigern Produkte mit einer geringen Verfärbung.

Die vorliegende Erfindung betrifft Verbindungen der Formel I oder II

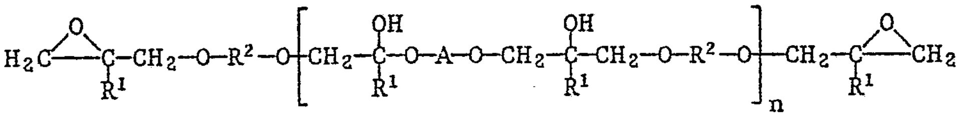 (I),

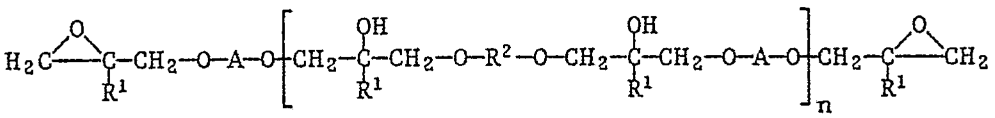 (II),

worin A ein Rest

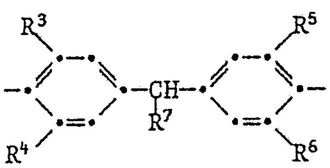

ist, $R^1$ Wasserstoff oder Methyl bedeutet,
$R^2$ der Rest eines aliphatischen, cycloaliphatischen, aromatischen oder araliphatischen Diols nach dem Entfernen beider Hydroxylgruppen ist,
$R^3$, $R^4$, $R^5$ und $R^6$ unabhängig voneinander Wasserstoff, $C_1$-$C_6$-Alkyl, Chlor oder Brom bedeuten,
$R^7$ ein Rest der Formel III, IV, V oder VI ist

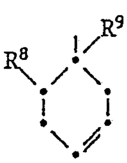 (III), 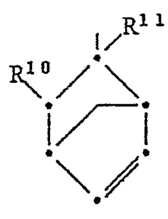 (IV), 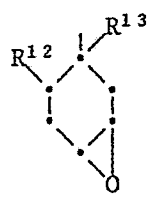 (V), 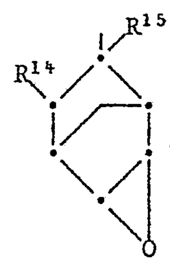 (VI),

$R^8$, $R^{10}$, $R^{12}$ und $R^{14}$ Wasserstoff, $C_1$-$C_6$-Alkyl oder Phenyl sind,
$R^9$, $R^{11}$, $R^{13}$ und $R^{15}$ Wasserstoff oder $C_1$-$C_6$-Alkyl bedeuten und der mittlere Wert von n (Zahlenmittel) eine Zahl von 1 bis 20 ist,
wobei die Reste $R^1$ bis $R^{15}$ innerhalb eines gegebenen Moleküls im Rahmen der gegebenen Definitionen unterschiedliche Bedeutungen annehmen können.
$R^1$ ist vorzugsweise Wasserstoff.

Leitet sich $R^2$ von einem aliphatischen Diol ab, so handelt es sich dabei um geradkettige oder

verzweigtkettige Alkylenreste, die gegebenenfalls durch Sauerstoff- oder Schwefelatome unterbrochen sein können, und die gegebenenfalls Substituenten tragen können.

Beispiele für Substituenten sind Chlor oder Brom.

Bevorzugt werden unsubstituierte geradkettige $C_2$-$C_{20}$-Alkylenreste. Beispiele dafür sind Ethylen, Tri-, Tetra-, Penta-, Hexa-, Hepta-, Octa-, Deca-, Dodeca-, Tetradeca-, Hexadeca-, Octadeca- oder Eicosamethylen.

Besonders bevorzugt wird Tetramethylen.

Das $R^2$ zugrundeliegende Diol kann aber auch ein Poly-(oxyalkylen)-glykol oder ein Poly-(thioalkylen)-glykol sein. Bevorzugt sind die sauerstoffhaltigen Derivate.

Beispiele dafür sind Poly-(ethylen)-glykol, Poly-(propylen)-glykol oder Poly-(butylen)-glykol mit 2-60 Monomereinheiten.

Leitet sich $R^2$ von einem cycloaliphatischen Diol ab, so handelt es sich dabei beispielsweise um ein Diol mit einem cycloaliphatischen Ring mit 5-7 Kohlenstoffatomen, der gegebenenfalls Teil einer aliphatischen Kette sein kann oder der gegebenenfalls Substituenten direkt am Ring trägt.

Beispiele für solche Reste sind Cyclopentylen, Cyclohexylen oder Cycloheptylen.

Besonders bevorzugt wird Cyclohexylen, insbesondere das 1,3- oder das 1,4-Cyclohexylen. Ebenfalls von Interesse ist das Hexahydroxylylen.

Basiert $R^2$ auf einem aromatischen Diol, so leitet sich dieser Rest bevorzugt von einem ein- oder zweikernigen Phenol ab. Bevorzugte Beispiele dafür sind 1,2-Phenylen oder insbesondere 1,3- oder 1,4-Phenylen sowie Reste der Formel VII

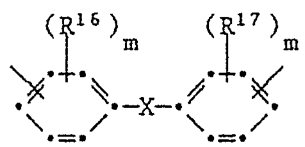

(VII),

worin die freien Bindungen vorzugsweise in 3- oder 4-Position zur Brücke X stehen und X eine direkte C-C-Bindung ist oder ausgewählt wird aus der Gruppe der Reste bestehend aus $-CH_2-$, $-CHCH_3-$, $-C(CH_3)_2-$, -O-, -S-, $-SO_2-$ oder -CO-, m eine ganze Zahl von 0 bis 4 ist, bevorzugt 0, 1 oder 2, ganz besonders bevorzugt 0, und $R^{16}$ und $R^{17}$ unabhängig voneinander $C_1$-$C_6$-Alkyl, Chlor oder Brom bedeuten.

Bevorzugte Reste $R^2$ sind Diphenylmethan-4,4′-diyl, Diphenylether-4,4′-diyl, Diphenylsulfon-4,4′-diyl und ganz besonders Diphenyl-2,2-propyliden-4,4′-diyl. Als araliphatischer Rest ist $R^2$ beispielsweise Xylylen.

$C_1$-$C_6$-Alkylreste $R^3$ bis $R^6$ und $R^8$ bis $R^{17}$ können geradkettig oder verzweigt sein. Beispiele dafür sind Methyl, Ethyl, n-Propyl, Isopropyl, n-Butyl, sek.-Butyl, tert.-Butyl, n-Pentyl oder n-Hexyl.

Bevorzugt werden geradkettige $C_1$-$C_4$-Alkylreste, insbesondere Methyl.

Ganz besonders bevorzugt werden Verbindungen der Formel I oder II, worin $R^2$ einen Rest der Formel VIIa darstellt

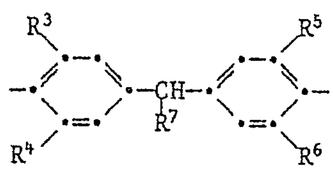

(VIIa),

und $R^3$, $R^4$, $R^5$, $R^6$ und $R^7$ die weiter oben definierte Bedeutung besitzen.

Die Reste $R^3$ bis $R^6$ und $R^8$ bis $R^{15}$ sind besonders bevorzugt Wasserstoff.

Die Verbindungen der Formel I oder II liegen in der Regel asl Gemisch von Komponenten unterschiedlicher Kettenlänge vor. Das mittlere Molekulargewicht (Zahlenmittel; ermittelt durch Gel-Permeationschromatographie) ist mindetens so gross wie das Molekulargewicht der entsprechenden reinen Verbindung mit $n = 1$.

Vorzugsweise ist der Mittelwert von n eine Zahl von 1 bis 10 und ganz besonders von 1 bis 5.

$R^7$ ist vorzugsweise ein Rest der Formel III oder der Formel IV. Weitere bevorzugte Reste $R^7$ sind die epoxidierten Typen der Formel VI oder insbesondere V, worin $R^{12}$, $R^{13}$, $R^{14}$ und $R^{15}$ Wasserstoff sind.

Die Verbindungen der Formel I leiten sich von Diphenolen der formel VIII ab

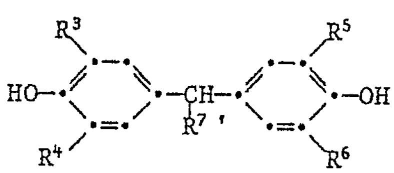

(VIII),

worin $R^3$, $R^4$, $R^5$ und $R^6$ die weiter oben definierten Bedeutungen besitzen und $R^{7\prime}$ ein Rest der Formel III oder

IV ist. Diese Verbindungen sind aus den oben erwähnten GB-Patenten 1,024,012 oder 1,024,013 bekannt oder können in Analogie zu den dort beschriebenen Verfahrensweisen hergestellt werden.

Die Erfindung betrifft aber auch ein Verfahren zur Herstellung von Verbindungen der Formel VIII, worin hohe Ausbeuten erzielt werden. Dieses Verfahren umfasst die Umsetzung von einem Aldehyd der Formel VIIIa

$R^{7'}$-CHO (VIIIa),

worin $R^{7'}$ die oben definierte Bedeutung besitzt mit einem 4- bis 8-fachen molaren Ueberschuss, bezogen auf den Aldehyd, eines Phenols der Formel VIIIb oder eines Gemisches dieser Phenole

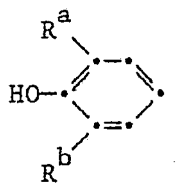

(VIIIb),

worin $R^a$ und $R^b$ ein weiter oben für $R^3$, $R^4$, $R^5$ und $R^6$ definierte Bedeutung besitzen; das Verfahren ist gekennzeichnet durch die Verwendung eines einkernigen aromatischen Kohlenwasserstoffs als Lösungsmittel und einer aromatischen Sulfonsäure als Katalysator. Falls gewünscht, kann zusätzlich noch eine Mercaptoverbindung anwesend sein, beispielsweise Thiomilchsäure, Mercaptoessigsäure oder Mercaptopropionsäure. Damit lässt sich eine weitere Steigerung der Ausbeute erzielen.

Die Reaktionstemperatur liegt in der Regel zwischen -10°C und der Siedetemperatur des jeweiligen Lösungsmittel(gemisches), bevorzugt zwischen 0-70°C.

Geeignete einkernige aromatische Kohlenwasserstoffe sind beispielsweise Benzol, Toluol, Ethylbenzol, Cumol, Xylol oder Chlorbenzol, insbesondere Toluol. Man kann auch Gemische einsetzen.

Geeignete aromatische Sulfonsäuren sind beispielsweise Benzol-, Toluol- oder Naphthalinsulfonsäuren, insbesondere jedoch Toluolsulfonsäure. Man kann auch Gemische einsetzen.

Die Diphenole der Formel VIII, worin $R^{7'}$ zusätzlich ein Rest der Formel V oder VI ist, sind auch als Härter für härtbare Epoxidharze einsetzbar, was ebenfalls einen Gegenstand der vorliegenden Erfindung darstellt.

Die Verbindungen der Formel I mit den ungesättigten Resten $R^7$ der Formel III oder IV lassen sich in an sich bekannter Weise durch Umsetzung von n Molen eines Diphenols der Formel VIII mit n + I Molen eins Diepoxids der Formel IX

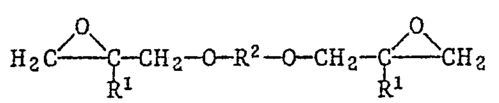

(IX),

worin n, $R^1$ und $R^2$ die weiter oben definierte Bedeutung besitzen, erhalten.

Die Verbindungen der Formel I mit den epoxidierten Resten $R^7$ der Formel V oder VI lassen sich erhalten, indem man eine Verbindung der Formel I mit ungesättigten Resten $R^7$ der Formel III oder IV in an sich bekannter Weise mit Persäure epoxidiert.

Die Epoxidierung mit Persäure kann auch so durchgeführt werden, dass nur ein Teil der ungesättigten Reste der Formel III oder IV in die entsprechende Epoxidverbindung übergeführt wird.

Die Verbindungen der Formel II leiten sich von Diglycidylethern der Formel X ab

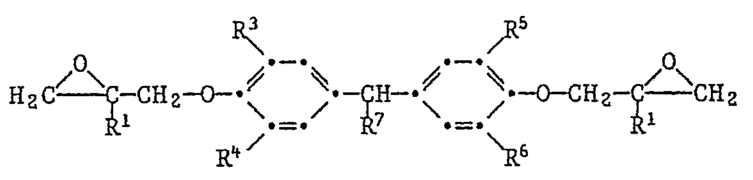

(X),

worin $R^1$, $R^3$, $R^4$, $R^5$, $R^6$ und $R^7$ die weiter oben definierte Bedeutung besitzen. Bei diesen Verbindungen handelt es sich also, je nach Natur des Restes $R^7$, um Di- oder Tri-Epoxidverbindungen.

Die Verbindungen der Formel X mit den Resten $R^7$ der Formel IV und VI sind neu und ebenfalls Gegenstand der vorliegenden Erfindung.

Die Di-Epoxidverbindungen der Formel X kann man aus den Diphenolen der Formel VIII in an sich bekannter Weise durch Umsetzung mit Epichlorhydrin oder mit β-Methylepichlorhydrin erhalten.

Die Tri-Epoxidverbindungen der Formel X lassen sich durch Umsetzung der Diglycidylether der Formel X enthaltend ungesättigte Reste $R^7$ der Formel III oder IV mit Persäure erhalten.

Die Verbindungen der Formel II mit ungesättigten Resten $R^7$ der Formel III oder IV lassen sich in an sich bekannter Weise durch Umsetzung von n Molen eines Diols der Formel XI

HO-$R^2$-OH (XI),

worin n und $R^2$ die weiter oben definierte Bedeutung besitzen, mit n + 1 Molen eines Diglycidylethers der

Formel X mit den Resten $R^7$ der Formeln III oder IV erhalten.

Die Verbindungen der Formel II mit den epoxidierten Resten $R^7$ der Formel V oder VI lassen sich erhalten, indem man

a) n + 1 Mole des Diglycidylethers der Formel X, worin $R^7$ Reste der Formel V oder VI sind, mit n Molen eines Diols der Formel XI in an sich bekannter Weise umsetzt oder

b) eine Verbindung der Formel II mit ungesättigten Resten $R^7$ der Formel III oder IV in an sich bekannter Weise mit Persäure epoxidiert.

Die Epoxidierung mit Persäure kann auch so durchgeführt werden, dass nur ein Teil der ungesättigten Reste der Formel III oder IV in die entsprechende Epoxidverbindungen übergeführt wird.

Bevorzugt setzt man n + l Mole des Diglycidylethers der Formel X mit n Molen des Diphenols der Formel VIII um und epoxidiert gegebenenfalls mit Persäure.

In einer weiteren bevorzugten Ausführungsform des Verfahrens setzt man n + l Mole des Diphenols der Formel VIII mit n Molen des Diglycidylethers der Formel X um und epoxidiert gegebenenfalls mit Persäure.

Die Vorverlängerung des Diglycidylethers IX mit dem Diphenol VIII oder des Diglycidylethers X mit dem Diol XI wird in Analogie zu bekannten Verfahren durchgeführt. Solche Vorverlängerungen werden z.B. bei der Synthese langkettiger Diglycidylether auf Bisphenol-Basis technisch durchgeführt und sind im "Epoxy-Handbook" von Lee und Neville, Kap. 2-9, beschrieben.

Die Epoxidierung olefinisch ungesättigter Verbindungen mittels Persäuren ist ebenfalls bekannt und wird z.B. im "Epoxy-Handbook" von Lee und Neville, Kap. 3, beschrieben.

Die vorverlängerten Verbindungen der Formel I oder II sowie die monomeren Ausgangsprodukte der Vorverlängerungsreaktion der Formel X lassen sich in Kombination mit Härtern als härtbare Gemische einsetzen.

Die Erfindung betrifft daher auch ein härtbares Gemisch enthaltend

a) mindestens eine Verbindung der Formel I oder II,

b) eine für die Härtung besagten Gemisches ausreichende Menge eines Epoxidhärters und

c) gegebenenfalls einen Härtungsbeschleuniger.

Ferner betrifft die Erfindung ein härtbares Gemisch enthaltend

a) mindestens eine Verbindung der Formel X, worin $R^7$ ein Rest der Formel IV oder VI ist,

b) eine für die Härtung besagten Gemisches ausreichende Menge eines Epoxidhärters und

c) gegebenenfalls einen Härtungsbeschleuniger.

Selbstverständlich lassen sich auch Gemische der Verbindungen I, II und/oder X einsetzen oder diese Verbindungen lassen sich in Kombination mit anderen bekannten Epoxidharzen verwenden.

Als Beispiele für zusätzliche Epoxidharze, die zusammen mit den Verbindungen I, II und/oder X eingesetzt werden können, wären zu erwähnen:

I) Polyglycidyl- und Poly-(β-methylglycidyl)-ester erhältlich durch Umsetzung einer Verbindung mit mindestens zwei Carboxylgruppen im Molekül und Epichlorhydrin bzw. Glycerindichlorhydrin bzw. β-Methylepichlorhydrin. Die Umsetzung erfolgt zweckmässig in der Gegenwart von Basen.

Als Verbindung mit mindestens zwei Carboxylgruppen im Molekül können aliphatische Polycarbonsäuren verwendet werden. Beispiele für diese Polycarbonsäuren sind Oxalsäure, Bernsteinsäure, Glutarsäure, Adipinsäure, Pimelinsäure, Korksäure, Azelainsäure, Sebazinsäure oder dimerisierte bzw. trimerisierte Linolsäure.

Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure.

Weiterhin können aromatische Polycarbonsäuren Verwendung finden, wie beispielsweise Phthalsäure, Isophthalsäure oder Terephthalsäure.

II) Polyglycidyl- oder Poly-(β-methylglycidyl)-ether erhältlich durch Umsetzung einer Verbindung mit mindestens zwei freien alkoholischen Hydroxygruppen und/oder phenolischen Hydroxygruppen und einem geeignet substituierten Epichlorhydrin unter alkalischen Bedingungen, oder in Anwesenheit eines sauren Katalysators und anschliessender Alkalibehandlung.

Ether dieses Typs leiten sich beispielsweise ab von acyclischen Alkoholen, wie Ethylenglykol, Diethylenglykol und höheren Poly(oxyethylen)-glykolen, Propan-1,2-diol oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit, Sorbit, sowie von Polyepichlorhydrinen.

Sie leiten sich aber auch beispielsweise ab von cycloaliphatischen Alkoholen wie 1,3- oder 1,4-Dihydroxycyclohexan, Bis-(4-hydroxycyclohexyl)-methan, 2,2-Bis(4-hydroxycyclohexyl)-propan oder 1,1-Bis-(hydroxymethyl)-cyclohex-3-en oder sie besitzen aromatische Kerne wie N,N-Bis-(2-hydroxyethyl)-anilin oder p,p'-Bis-(2-hydroxyethylamino)-diphenylmethan.

Die Epoxidverbindungen können sich auch von einkernigen Phenolen ableiten, wie beispielsweise von Resorcin oder Hydrochinon; oder sie basieren auf mehrkernigen Phenolen wie beispielsweise auf Bis-(4-hydroxyphenyl)-methan, 4,4'-Dihydroxydiphenyl, Bis(4-hydroxyphenyl)-sulfon, 1,1,2,2-Tetrakis-(4-hydroxyphenyl)-ethan, 2,2-Bis-(4-hydroxyphenyl)-propan, 2,2-Bis-(3,5-dibrom-4-hydroxyphenyl)- propan sowie auf Novolaken erhältlich durch Kondensation von Aldehyden, wie beispielsweise Formaldehyd, Acetaldehyd, Chloral oder Furfuraldehyd mit Phenolen wie Phenol, oder mit Phenolen, die im Kern mit Chloratomen oder $C_1$-$C_9$Alkylgruppen substituiert sind, wie beispielsweise 4-Chlorphenol, 2-Methylphenol oder 4-tert.Butylphenol oder erhältlich durch Kondensation mit Bisphenolen, so wie oben beschrieben.

III) Poly-(N-glycidyl) Verbindungen die beispielsweise erhältlich sind durch Dehydrochlorierung der Reaktionsprodukte von Epichlorhydrin mit Aminen, die mindestens zwei Aminowasserstoffatome enthalten. Bei diesen Aminen handelt es sich zum Beispiel um Anilin, n-Butylamin, Bis-(4-aminophenyl)-methan, m-Xylylendiamin oder Bis-(4-methylaminophenyl)-methan.

Zu den Poly-(N-glycidyl) Verbindungen zählen aber auch Triglycidylisocyanurat, N,N'-Diglycidylderivate von Cycloalkylenharnstoffen, wie Ethylenharnstoff oder 1,3-Propylenharnstoff, und N,N'-Diglycidylderivate von Hydantoinen, wie von 5,5-Dimethylhydantoin.

IV) Beispiele für Poly-(S-glycidyl) Verbindungen sind Di-S-glycidylderivate, die sich von Dithiolen, wie beispielsweise Ethan-1,2-dithiol oder Bis-(4-merkaptomethylphenyl)-ether ableiten.

V) Beispiele für cycloaliphatische Epoxidharze sind Bis-(2,3-epoxicyclopentyl)-ether, 2,3-Epoxicyclopentylglycidylether oder 1,2-Bis-(2,3-epoxycyclopentyloxy)-ethan.

Es lassen sich aber auch Epoxidharze verwenden, bei denen die 1,2-Epoxidgruppen an unterschiedliche Heteroatome bzw. funktionelle Gruppen gebunden sind; zu diesen Verbindungen zählen beispielsweise das N,N,O-Triglycidylderivat des 4-Aminophenols, der Glycidylether-glycidylester der Salicylsäure, N-Glycidyl-N'-(2-glycidyloxypropyl)-5,5-dimethylhydantoin oder 2-Glycidyloxy-1,3-bis-(5,5-dimethyl-1-glycidylhydantoin-3-yl)-propan.

Als Epoxidhärtungsmittel b) kommen saure, basische oder katalytische Härter in Frage. Dazu zählen beispielsweise Amine oder Amide, wie aliphatische, cycloaliphatische oder aromatische, primäre, sekundäre oder tertiäre Amine, beispielsweise Hexamethylendiamin, N,N-Diethylpropylendiamin, Bis-(4-aminocyclohexyl)-methan, 3,5,5-Trimethyl-3-(aminomethyl)-cyclohexylamin ('Isophorondiamin'), 2,4,6-Tris-(dimethylaminomethyl)-phenol, p-Phenylendiamin oder Bis-(4-aminophenyl)-methan; oder Polyamide, beispielsweise solche aus aliphatischen Polyaminen und di- oder trimerisierten ungesättigten Fettsäuren; oder mehrwertige Phenole, wie beispielsweise Resorcin, 2,2-Bis-(4-hydroxyphenyl)-propan oder Phenol-Formaldehydharze (Phenol-Novolake); oder Bortrifluorid und seine Komplexe mit organischen Verbindungen, wie beispielsweise $BF_3$-Ether-Komplexe oder $BF_3$-Amin-Komplexe; oder mehrbasische Carbonsäuren und deren Anhydride, wie beispielsweise Phthalsäureanhydrid, Tetrahydrophthalsäureanhydrid oder Hexahydrophthalsäureanhydrid oder die entsprechenden Säuren.

Man kann bei der Härtung ausserdem Härtungsbeschleuniger c) einsetzen; solche Beschleuniger sind z.B. tertiäre Amine, deren Salze oder quaternäre Ammoniumverbindungen, z.B. Benzyldimethylamin, 2,4,6-Tris(dimethylaminomethyl)phenol, 1-Methylimidazol, 2-Ethyl-4-methylimidazol, 4-Aminopyridin, Tripentylammoniumphenolat oder Tetramethylammoniumchlorid; oder Alkalimetallalkoholate, wie z.B. Na-Alkoholate von 2,4-Dihydroxy-3-hydroxymethylpentan.

Derartige härtbare Gemische können ferner geeignete Weichmacher, wie Dibutylphthalat, Dioctylphthalat oder Trikresylphosphat, oder auch reaktive Verdünner, wie Phenyl- oder Kresylglycidylether, Butandioldiglycidylether oder Hexahydrophthalsäurediglycidylester enthalten.

Schliesslich können die härtbaren Gemische vor der Härtung in irgendeiner Phase mit Streck-, Füll- und Verstärkungsmitteln, wie beispielsweise Steinkohlenteer, Bitumen, Textilfasern, Glasfasern, Asbestfasern, Borfasern, Kohlenstoff-Fasern, mineralischen Sili katen, Glimmer, Quarzmehl, Aluminiumoxidhydrat, Bentoniten, Kaolin, Kieselsäureaerogel oder Metallpulvern, z.B. Aluminiumpulver oder Eisenpulver, ferner mit Pigmenten und Farbstoffen, wie Russ, Oxidfarben, Titandioxid, u.a. versetzt werden. Man kann den härtbaren Gemischen ferner auch andere übliche Zusätze. z.B. Flammschutzmittel, wie Antimontrioxid, Thixotropiemittel, Verlaufmittel ("flow control agents"), wie Silicone, Wachse oder Stearate (welche zum Teil auch als Formtrennmittel Anwendung finden), zusetzen.

Die Herstellung der erfindungsgemässen härtbaren Mischungen kann in üblicher Weise mit Hilfe bekannter Mischaggregate (Rührer, Kneter, Walzen etc.) erfolgen.

Die erfindungsgemässen härtbaren Epoxidharzmischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepasster Formulierung, im ungefüllten oder gefüllten Zustand, als Anstrichmittel, Lacke, wie Sinterpulverlacke, als Pressmassen, Tauchharze, Giessharze, Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate verwendet werden.

Bevorzugt sind sie als Sinterpulverlacke, Imprägnier- und Laminierharze, insbesondere als Imprägnier- und Laminierharze, verwendbar.

Die Härtung der erfindungsgemässen glycidylgruppenhaltigen Copolymeren wird zweckmässig im Temperaturintervall von 50°C bis 300°C, bevorzugt von 80-250°C, durchgeführt.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der Epoxi-Komponente a), dem Härter b) und dem gegebenenfalls anwesenden Beschleuniger c) erhalten wird. Ein derartiges Vorkondensat kann z.B. zur Herstellung von "Prepregs", Pressmassen oder Sinterpulvern dienen.

Der Ausdruck "Härten", wie er hier gebraucht wird, bedeutet die Umwandlung der löslichen, entweder flüssigen oder schmelzbaren Polyepoxide in feste, unlösliche und unschmelzbare, dreidimensional vernetzte

Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen und Verklebungen.

Die folgenden Beispiele erläutern die Erfindung näher.

## Herstellungsbeispiele

### Herstellung der vorverlängerten Epoxidharze

### Beispiel 1

2

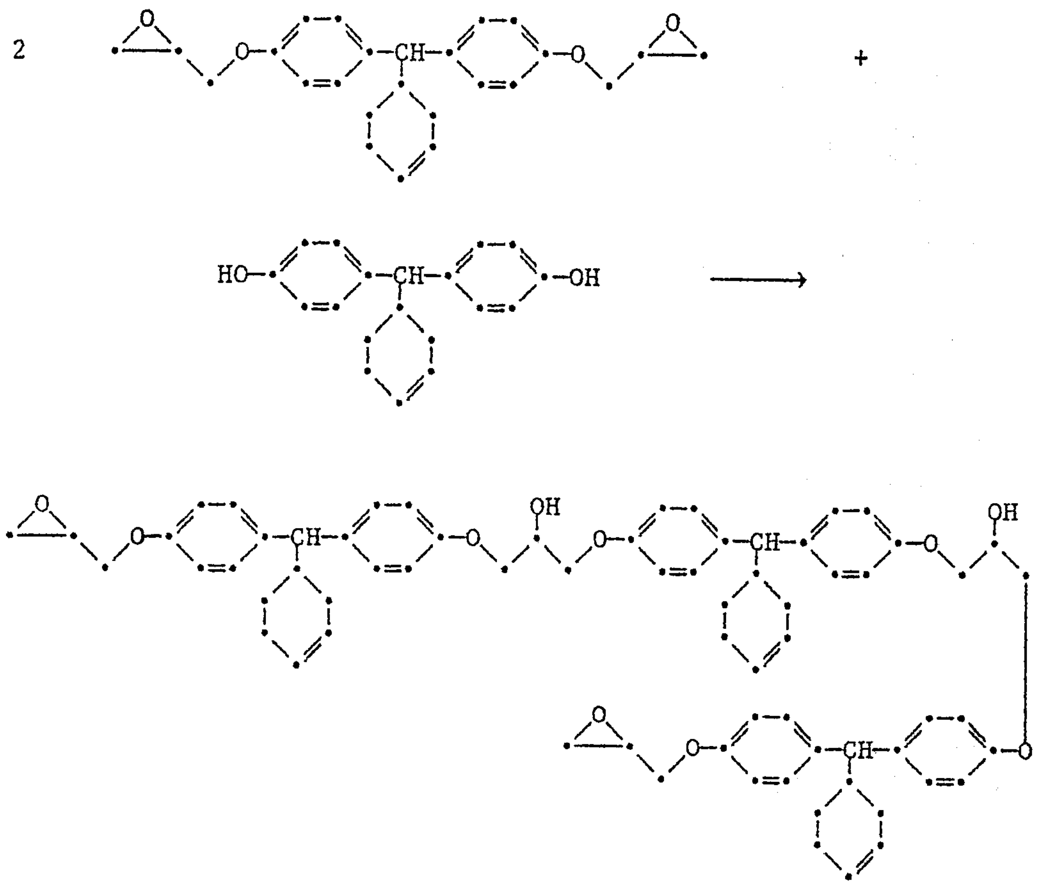

+

In einem 750 ml Sulfierkolben, versehen mit Ankerrührer, Rückflusskühler und Tropftrichter werden 502,60 g (1,20 Mol) Diglycidylether von 4,4′-(Cyclohex-3-en-1-yl-methylen)-diphenol (4,78 g Aequ./kg) und 0,31 g 2-Phenylimidazol
vorgelegt und auf 150° C Innentemperatur aufgeheizt. Innerhalb ca. 1 h fügt man portionenweise unter Rühren 168,22 g (0,60 Mol) 4,4′-(Cyclohex-3-en-1-yl-methylen)-diphenol zu, wobei die Temperatur unter 170° C gehalten wird. Man lässt weitere 2 h bei 150°-160° C rühren, giesst die heisse Schmelze auf ein Blech, kühlt auf Raumtemperatur und Pulverisiert das erstarrte Harz. Man gewinnt 632 g schwach gefärbtes Festharz.

Elementaranalyse:
berechnet (%): C, 77,79 H 7,19
gefunden (%): C 76,99 H, 7,21

Epoxi-Wert:
berechnet: 1,88 Aequ./kg
gefunden: 1,47 Aequ./kg

GPC (Polystyrol Standards)
4 Hauptpeaks
$\bar{M}_w = 2590$
$\bar{M}_n = 1227$

Beispiel 2

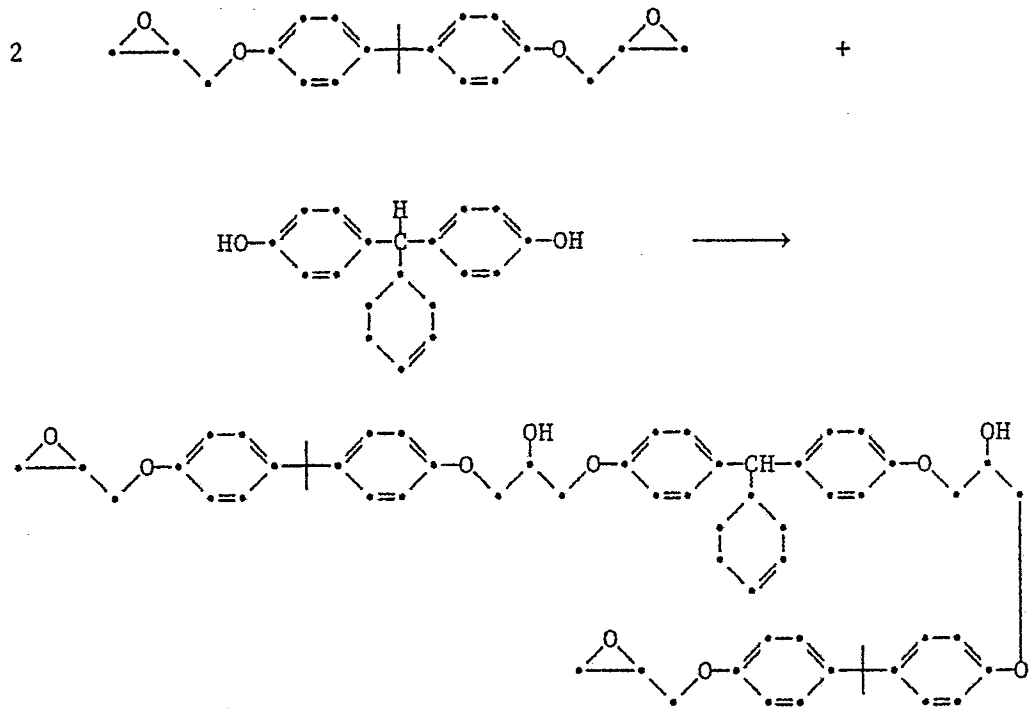


In einem 750 ml Sulfierkolben werden gemäss Verfahren des Beispiels 1
416,80 g (1,20 Mol) Bisphenol-A-Diglycidylether
168,21 g (0,60 Mol) 4,4'-(Cyclohex-3-en-1-yl-methylen)diphenol
0.48 g 2-Phenylimidazol
umgesetzt. Man erhält 561 g schwach gefärbtes Festharz.

Elementaranalyse:
berechnet (%): C 76,22 H 7,13
gefunden (%): C 75,88 H 7,11

Epoxi-Wert:
berechnet: 2,08 Val Aequ./kg
gefunden: 1,79 Val Aequ./kg

GPC (Polystyrol Standards)
4 Hauptpeaks
$\bar{M}_w = 4178$
$\bar{M}_n = 1354$

Beispiel 3

Epoxidation des Produktes aus Beispiel 2

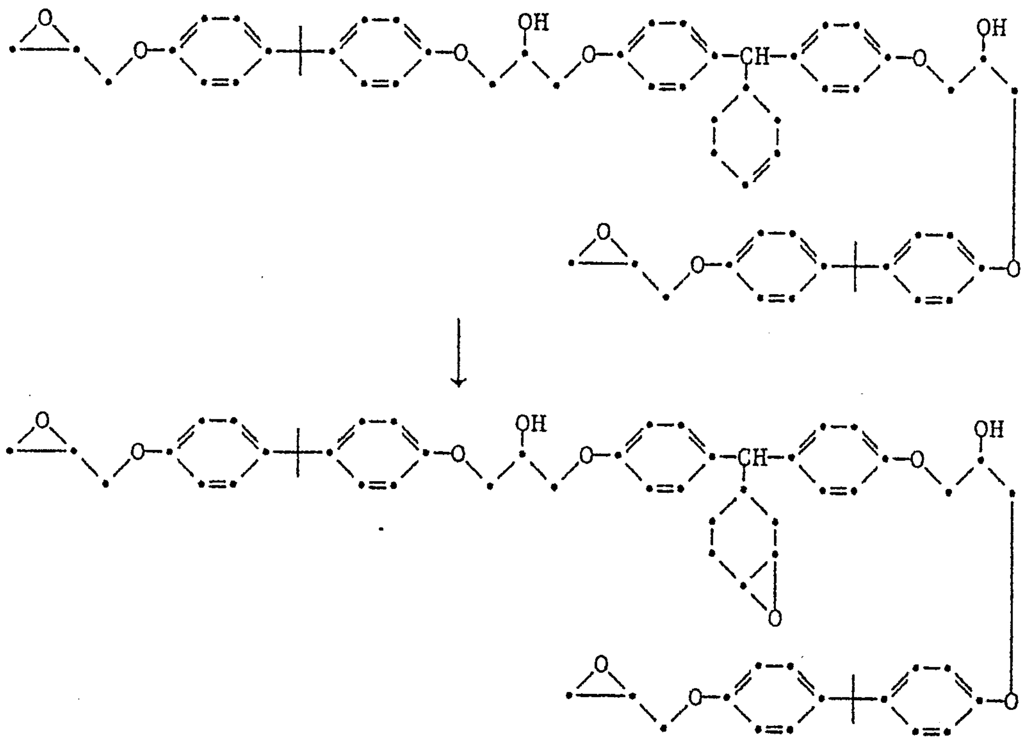

In einem 4.0 l Sulfierkolben mit Thermometer, Rührer, Kühler und Impulsomat bzw. Dosimat werden 528,66 g (0.55 Mol) des Produktes aus Beispiel 2 in 2,0 l Chloroform vorgelegt. Innerhalb 1[1]/2 Std. werden 159,60 g (0,842 Mol) 40%iger Peressigsäure bei 35°-37°C zugetropft, wobei der pH mit 20%iger NaOH stabil gehalten wird (pH 4,0). Nach Beenden des Zutropfens wird das Reaktionsgemisch noch während ca. 4 Std. ausreagieren gelassen, anschliessend mit ca. 500 ml Chloroform verdünnt, mit 1 l 1N NaOH und 3 x 1 l Wasser neutral gewaschen, über $Na_2SO_4$ und $Na_2SO_3$ getrocknet und eingeengt. Nach dem Einengen und vollständigen Trocknen resultieren 487,62 g (90,73 % der Theorie) eines gelblichen Pulvers von Epoxidgehalt 2,59 Aeq./kg (84 % der Theorie).

Beispiel 4

Epoxidation des Produktes aus Beispiel 1

585,97 g (0,55 Mol) des Produktes aus Beispiel 1 und 1200 ml Chloroform werden in einem 2,5 l Sulfierkolben vorgelegt und auf 45°-50°C erwärmt. Anschliessend werden innerhalb 5 Std. unter kontrolliertem pH (pH 4,0) 478,50 g (2,53 Mol) 40%ige Peressigsäure bei 45°-50°C zugetropft. Nach Beendigen des Zutropfens wird das Reaktionsgemisch noch während ca. 2 Std. ausreagieren gelassen, anschliessend mit ca. 500 ml Chloroform verdünnt, und mit 1 l 1N NaOH sowie mehrmals mit 1 l $H_2O$ gewaschen, über $Na_2SO_4$ getrocknet und mit $Na_2SO_3$ peroxidfrei gemacht, filtriert und eingeengt. Nach Einengen am Hochvakuum (ca. 13 Pa) und Trocknen resultieren 434,4 g eines gelblichen Pulvers vom Epoxidgehalt 3,34 Aeq./kg (74,39 % der Theorie).

Beispiel 5

2 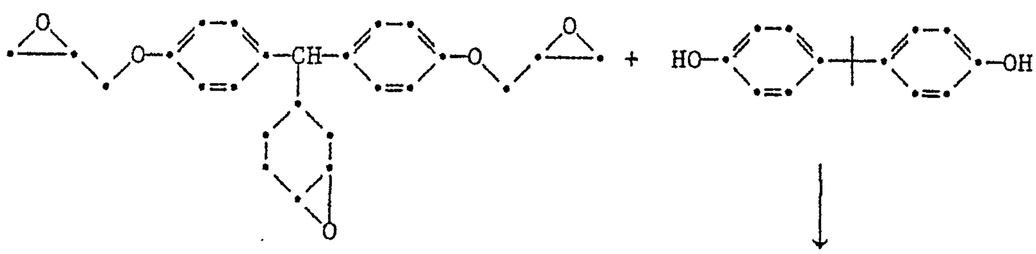

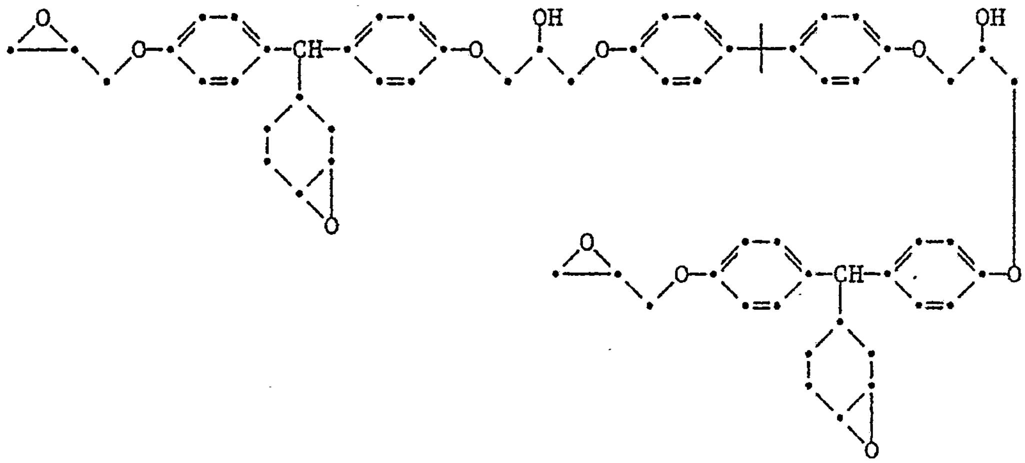

In einem 100 ml-Sulfierkolben, versehen mit Ankerrührer, Thermometer und Rückflusskühler werden 47,17 g (0,10 Mol) Diglycidylether von 4,4'-(3,4-Epoxicyclohex-1-ylmethylen)-diphenol (Epoxidgruppengehalt = 6,37 Aeq./kg)
11,42 g (0,05 Mol) Bisphenol A
0,04 g 2-Phenylimidazol
auf 150-160° C Innentemperatur aufgeheizt und unter Rühren 2 1/2 h bei dieser Temperatur gehalten. Die klare gelbliche Schmelze wird auf ein Blech gegossen und nach dem Erstarren pulverisiert. Man erhält 56,0 g schwach gelbliches Festharz.

Elementaranalyse:
berechnet (%): C, 74,69 H 6,94
gefunden (%): C, 72,39 H 6,89

Epoxidgruppengehalt:
berechnet: 3,41 Aeq/kg
gefunden: 2,94 Aeq/kg (86 % d.Th.)

GPC (Polystyrol Standards)
$\bar{M}_w = 6113$
$\bar{M}_n = 1351$

Beispiel 6:

2 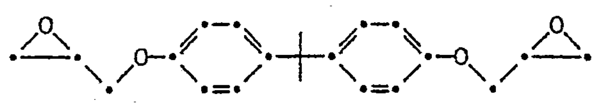 +

1 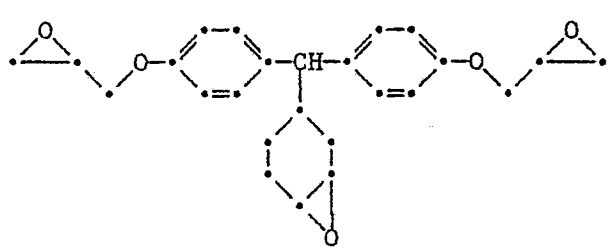 +

1 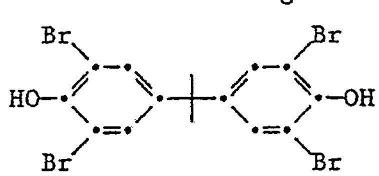

In einem 250 ml Sulfierkolben, versehen mit Ankerrührer, Thermometer, Rückflusskühler und $N_2$-Inertgasschutz werden
30,80 g Tetrabrombisphenol A (Hydroxylgruppengehalt = 3,68 Aeq./kg)

42,50 g Bisphenol-A-diglycidylether (Epoxidgruppengehalt = 5,34 Aeq./kg)
26,70 g Diglycidylether von 4,4'-(3,4-Epoxycyclohex-1-ylmethylen)diphenol (Epoxidgruppengehalt = 6,37 Aeq./kg)
0,08 g 2-Phenylimidazol
auf 160°C Innentemperatur erhitzt und unter Rühren 6 1/2 h bei dieser Temperatur gehalten. Die klare, gelbliche Schmelze wird auf ein Blech gegossen und nach dem Erstarren pulverisiert. Man erhält 93,96 g gelbliches Festhärz.

Epoxidgruppengehalt
berechnet: 2,84 Aeq./kg
gefunden: 2,38 Aeq./kg (83 % d.Th.)

GPC (Polystyrol-Standards)
$\bar{M}_w = 2258$
$\bar{M}_n = 932$

Herstellung der Zwischenprodukte der Vorverlängerungsreaktion

Beispiel a

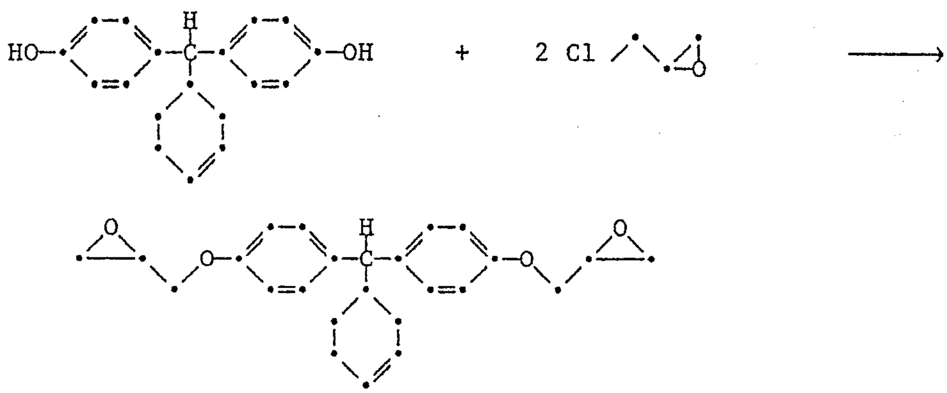

In einem 2,5 l-Sulfierkolben, versehen mit Rührer, Rückflusskühler, Thermometer und Tropftrichter werden
280,40 g (1,0 Mol) 4,4'-(Cyclohex-3-en-1-yl-methylen)-diphenol
1110,40 g (12,0 Mol) Epichlorhydrin
150,00 g Isopropanol
vorgelegt und unter Rühren auf 70°C Innentemperatur aufgeheizt. Innerhalb ca. 2 h fügt man tropfenweise 224,00 g (2,80 Mol) einer 50%iger NaOH-Lösung zu, wobei die Temperatur bei 70°C-75°C gehalten wird. Nach der Zugabe lässt man noch 2 h bei 70°C weiterrühren, kühlt auf 20°C und wäscht das Reaktionsprodukt (etwas schmierige Suspension) 2x mit je 750 ml 5%iger $NaHSO_4$-Lösung und 1x mit 1000 ml Wasser. Die organische Phase wird über Natriumsulfat getrocknet, filtriert und im Vakuum eingedampft. Nach der Trocknung im Vakuum bei 120°C/l,3 Pa (3 h) erhält man 368,9 g eines klaren, schwach gefärbten Harzes.

Elementaranalyse:
berechnet (%): C 76,50 H 7,19
gefunden (%): C 75,67 H 7,24

Epoxi-Wert:
berechnet: 5,1 Aequ./kg
gefunden: 4,7 Aequ./kg

Beispiel b

Epoxidation des Produktes aus Beispiel a

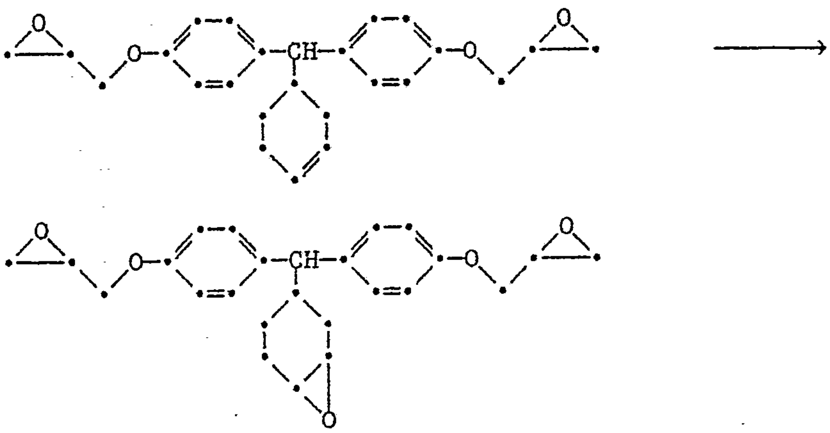

In einem 2,5 l Sulfierkolben mit Rührer, Thermometer, Kühler, pH-Meter, pH-Elektrode, Dosimat und Impulsomat werden 333,63 g (0,850 Mol) des Diglycidylethers aus Beispiel a vom Epoxigehalt 4,305 Aeq./kg zusammen mit 680 ml Chloroform vorgelegt. Bei einer Temperatur von 40°-50° C werden 246,50 g (1,292 Mol) 40%ige Peressigsäure innerhalb 4 Std. zugetropft, wobei das pH immer auf pH 4,0 stabil gehalten wird. Nach Beenden des Zutropfens wird das Reaktionsgemisch noch während 1 Std. nachreagiert, anschliesend wird die wässrige Phase abgetrennt, die organische Phase mit 2x 500 ml Wasser und 1x 250 ml $NaHCO_3$ neutral gestellt, über $Na_2SO_4$ getrocknet bzw. mit $Na_2SO_3$ peroxidfrei gemacht, filtriert und am Vakuum eingeengt (50°-70° C/665-1330 Pa).

Nach dem Trocknen verbleiben 316,0 g (91,01 % der Theorie) eines farblosen Pulvers von Epoxidgehalt 6,36 Aeq./kg und einer Viskosität von 5835 mPas bei 80° C. $M_n/M_w$ 428/449.

IR (Film): 3500-3200, 3000-2800, 1600, 1520, 1240, 1040 820 $cm^{-1}$.

NMR ($CDCl_3$): 0,8-2.5 m 9H (Cyclohexyl-H), 2,5-4,0 m 11H (Glycidyl H2 Methin-H), 6,8-7,2 d x d 8H (arom. H).

Herstellung von Cyclohex-3-en-l-ylmethylendiphenol

Beispiel A

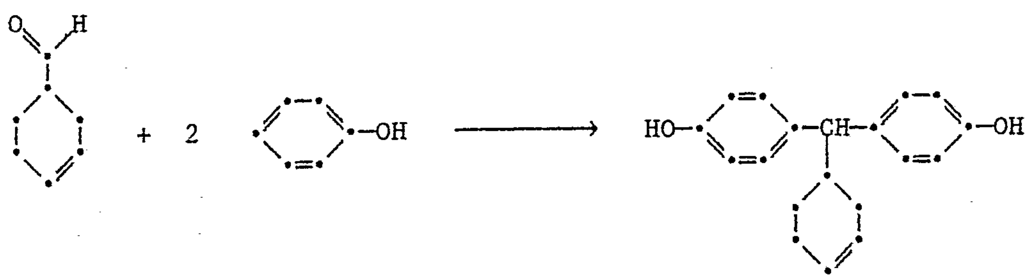

In einem 4 l-Sulfierkolben, versehen mit Rührer, Rückflusskühler, Tropftrichter, Thermometer werden unter Inertgasschutz:

1129,3 g (12,0 Mol) Phenol
10,0 ml 3-Mercaptopropionsäure
76,0 g (0,40 Mol) Toluol-4-sulfonsäuremonohydrat
600,0 ml Toluol

vorgelegt und zu einer Lösung verrührt. Mit einem Eisbad kühlt man auf +15° C Innentemperatur und tropft innert 1 Stunde 220,2 g (2,0 Mol) 1,2,5,6-Tetrahydrobenzaldehyd unter Kühlung zu, so dass die Innentemperatur bei ca. +15° C bleibt. Das Kühlbad wird entfernt und man lässt weitere 6 h bei Raumtemperatur rühren. Die gebildete Suspension wird abfiltriert und der Filterrückstand bei 80° C im Vakuum getrocknet.

Man erhält 424,8 g (Ausbeute = 75,7 % ber. auf eingesetzten Tetrahydrobenzaldehyd) rötlich gefärbtes Cyclohexenyl-methylendiphenol. Nach Umkristallisieren aus 1300 ml Isopropanol verbleiben 319,6 g farbloses Produkt (57 % Ausbeute bezogen auf Tetrahydrobenzaldehyd).

Smp.: 215-216°

Elementaranalyse (umkristallisiertes Produkt)
berechnet (%) C 81,40 H 7,19
gefunden (%) C 81,02 H 7,21

Applikationsbeispiele

I. Phenolische Härtung (mit Kresolnovolak)

Die folgenden Formulierungen werden als 65%ige Lösungen in DMF auf Aluminiumbleche beschichtet und getrocknet. Die derart erhaltenen Filme weisen eine Trockenschichtdicke von ca. 50 µm auf. Nach dem Aushärten bei 180° während 1 h werden die Glastemperatur sowie die Chemikalienbeständigkeit bestimmt.

| Versuch | A | B | C |
|---|---|---|---|
| Formulierung<br><br><br><br><br>Lösung in DMF | Produkt gemäss Bsp.4 100T<br>1) Novolak 31T<br>2) EMI-2,4 1,4T<br>65 % | Produkt gemäss Bsp.3 100T<br>Novolak 44T<br>EMI-2,4 2,0T<br>65 % | Produkt gemäss Bsp.5 100T<br>Novolak 35T<br>EMI-2,4 1,6T<br>65 % |
| Härtung | 1 h 180°C | 1 h 180°C | 1 h 180°C |
| 3) $T_G$ | 174°C | 185°C | 170°C |
| 4) Reibtest (20x) Aceton | 0 | 0 | 0 |
| 5) Chemikalienbeständigkeit:<br>5N NaOH<br>5N $H_2SO_4$ | <br>0<br>0 | <br>0<br>0 | <br>0<br>0 |

Wie aus obiger Tabelle ersichtlich, zeigen die ausgehärteten Harze hohe Glastemperaturen und ausgezeichnete Resistenz gegen Chemikalien.

1) Kresolnovolak, Hydroxylgruppengehalt = 8,3 Aeq./kg
2) 2-Ethyl-4-methylimidazol
3) Glastemperatur aus thermomechanischer Analyse.
4) Acetonreibtest: 0-5 0 = Schicht nicht angegriffen
5 = Schicht weggelöst
5) Chemikalienbeständigkeit: Einwirkung von 1 Tropfen während 1 h auf die Schicht (bei RT), dann Kratzen mit Spatel
0 - 5 0 = Schicht nicht angegriffen
5 = Schicht weggelöst

II. Pulverlack

Probenherstellung: Die Formulierkomponenten gemäss folgender Tabelle werden gemahlen und gut gemischt. Das Pulver wird auf eine vorgewärmte Aluminiumplatte (180° C) aufgebracht, so dass eine Filmdicke von 40-60 µm resultiert. Man härtet 30 Minuten bei 180° C und bestimmt dann die in der Tabelle aufgeführten Eigenschaften.

Wie man sieht, ergeben die ausgehärteten Harze vergilbungsfreie Beschichtungen mit guten mechanischen Eigenschaften und guter Chemikalienbeständigkeit. Bemerkenswert ist die hohe Reaktivität der Harzmischung, die sich in einer kurzen Gelierzeit niederschlägt.

Erklärungen:

1) Uralac® P 3400 = fester, saurer, gesättigter Polyester der Firma Scado.

2) Formulierter Beschleuniger auf Basis von einem gesättigten, carboxyl-terminierten Polyester und $C_{12}$-$C_{16}$-Alkyltrimethylammoniumbromid.

Tabelle

| Versuch | D | E | F |
|---|---|---|---|
| Formulierung | Produkt gemäss<br>Bsp.4 145 T.<br>Uralac 3400 [1]) 855 T.<br>Beschleuniger [2]) 20 T. | Produkt gemäss<br>Bsp.3 195 T.<br>Uralac 3400 [1]) 805 T.<br>Beschleuniger [2]) 20 T. | Produkt gemäss<br>Bsp.5 175 T.<br>Uralac 3400 [1]) 825 T<br>Beschleuniger [2]) 20 T |
| Gelierzeit bei 180°C (min) | 2 | 2 | 2 |
| Härtung (°C)<br>(min) | 180<br>30 | 180<br>30 | 180<br>30 |
| Vergilbung | farblos | farblos | farblos |
| Schlagerichsen (cm•kg) | <10 | >180 | >180 |
| Erichsentiefe (mm) | <1 | >10 | >10 |
| Haftung (0-5) (Gitterschnitt) | Gt 0 | Gt 0 | Gt 0 |
| Acetontest (0-5) (1 min) | 3-4 | 2-3 | 2-3 |
| König-Härte (sec) | 208 | 218 | 216 |
| Tg (°C) (DSC) | 84 | 85 | 88 |
| Differential-Thermo-gravimetrie; 10°C/min<br>T(-5 %) (°C)<br>T(-10 %) (°C)<br>T(-50 %) (°C) | <br>370<br>395<br>460 | <br>370<br>390<br>450 | <br>365<br>390<br>460 |

III. Laminat (Leiterplatte)

Vom Produkt aus Beispiel 6 wird eine 75 %ige Lösung in Methylethylketon hergestellt und damit eine Imprägnierlösung der folgenden Zusammensetzung formuliert.

133,0 Teile 75 %ige Lösung vom Produkt aus Beispiel 6
37,7 Teile 10 %ige Lösung von Dicyandiamid in Methylglycol
3,9 Teile 5 %ige Lösung von Benzylmethylamin in Methylglycol
29,0 Teile Methylglycol

Mit dieser Lösung wird Glasgewebe (Typ 7628 CS, Finish Z 6040) imprägniert und bei 170°C während 4 1/2 Minuten im Umluftofen getrocknet. Die Prepregs werden anschliessend zusammen mit Kupferfolie bei 170°C während 2 Stunden mit einem Druck von 20-40 kg/cm$^2$ zu Laminaten mit folgenden Eigenschaften verpresst.

Glastemperatur $T_G$ (DSC): 160°C
N-Methylpyrrolidon-Aufnahme: 0,0 %
Pressure-cooker-test (1h): bestanden
Flammbarkeit (UL-94): V-O

**Patentansprüche**

1. Verbindungen der Formeln I oder II

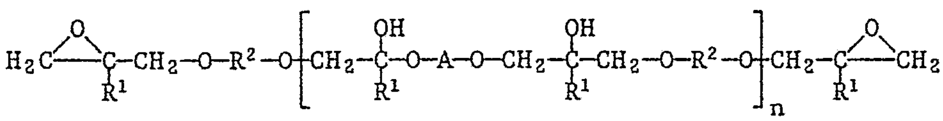 (I),

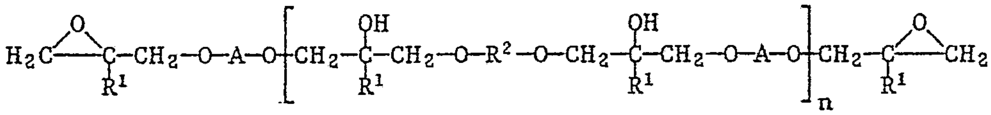 (II),

worin A ein Rest

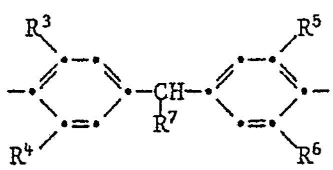

ist, $R^1$ Wasserstoff oder Methyl bedeutet,
$R^2$ der Rest eines aliphatischen, cycloaliphatischen, aromatischen oder araliphatischen Diols nach dem Entfernen beider Hydroxylgruppen ist,
$R^3$, $R^4$, $R^5$ und $R^6$ unabhängig voneinander Wasserstoff, $C_1$-$C_6$-Alkyl, Chlor oder Brom bedeuten,
$R^7$ ein Rest der Formel III, IV, V oder VI ist

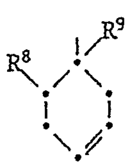 (III), 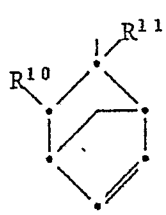 (IV), 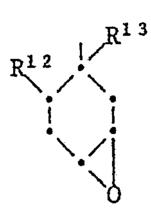 (V), 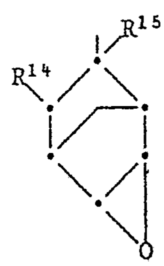 (VI),

$R^8$, $R^{10}$, $R^{12}$ und $R^{14}$ Wasserstoff, $C_1$-$C_6$-Alkyl oder Phenyl sind,
$R^9$, $R^{11}$, $R^{13}$ und $R^{15}$ Wasserstoff oder $C_1$-$C_6$-Alkyl bedeuten und der mittlere Wert von n (Zahlenmittel) eine Zahl von 1 bis 20 ist,
wobei die Reste $R^1$ bis $R^{15}$ innerhalb eines gegebenen Moleküls im Rahmen der gegebenen Definitionen unterschiedliche Bedeutungen annehmen können.

2. Verbindungen der Formeln I oder II gemäss Anspruch 1, worin $R^1$ Wasserstoff ist.

3. Verbindungen der Formeln I oder II gemäss Anspruch 1, worin $R^2$ 1,2-, 1,3- oder 1,4-Phenylen ist oder Reste der Formel VII darstellt

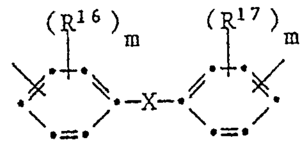

(VII),

worin X eine direkte C-C-Bindung ist oder ausgewählt wird aus der Gruppe der Reste bestehend aus $-CH_2-$, $-CHCH_3-$, $-C(CH_3)_2-$, -O-, -S-, $-SO_2-$ oder -CO-, m eine ganze Zahl von 0 bis 4 ist und $R^{16}$ und $R^{17}$ unabhängig voneinander $C_1$-$C_6$-Alkyl, Chlor oder Brom bedeuten.

4. Verbindungen der Formeln I oder II gemäss Anspruch 1, worin $R^2$ einen Rest der Formel VIIa darstellt

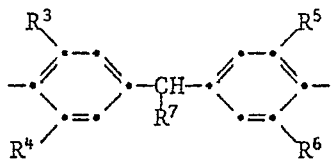

(VIIa),

und $R^3$, $R^4$, $R^5$, $R^6$ und $R^7$ die in Anspruch 1 definierte Bedeutung besitzen.

5. Verbindungen der Formel I oder II gemass Anspruch 1, worin der Mittelwert von n eine Zahl von 1 bis 10 ist.

6. Verbindungen der Formeln I oder II gemäss Anspruch 1, worin $R^7$ ein Rest der Formel III oder IV ist.

7. Verbindungen der Formeln I oder II gemäss Anspruch 1, worin $R^7$ ein Rest der Formel V oder VI ist, worin $R^{12}$, $R^{13}$, $R^{14}$ und $R^{15}$ Wasserstoff sind.

8. Verfahren zur Herstellung von Verbindungen der Formel VIII

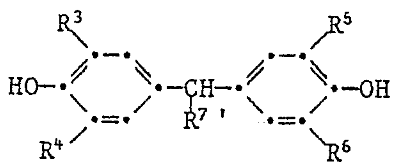

(VIII),

worin $R^3$, $R^4$, $R^5$ und $R^6$ die in Anspruch 1 definierten Bedeutungen besitzen und $R^{7'}$ ein Rest der Formeln III oder IV gemäss Anspruch 1 ist durch Umsetzung von einem Aldehyd der Formel VIIIa

$R^{7'}$-CHO (VIIIa),

worin $R^{7'}$ die oben definierte Bedeutung besitzt, mit einem 4- bis 8-fachen molaren Ueberschuss, bezogen auf den Aldehyd, eines Phenols der Formel VIIIb oder eines Gemisches dieser Phenole

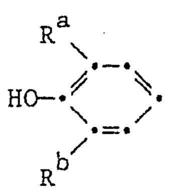

(VIIIb),

worin $R^a$ und $R^b$ die Bedeutung von $R^3$, $R^4$, $R^5$ und $R^6$ gemäss Anspruch 1 besitzen, durch gekennzeichnet, dass man einen einkernigen aromatischen Kohlenwasserstoff als Lösungsmittel und eine aromatische Sulfonsäure als Katalysator verwendet.

9. Verbindungen der Formel X

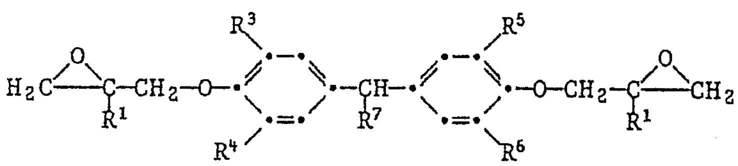

(X),

worin $R^1$, $R^3$, $R^4$, $R^5$ und $R^6$ die in Anspruch 1 definierte Bedeutung besitzen und $R^7$ ein Rest der Formeln IV oder VI gemäss Anspruch 1 ist.

10. Härtbares Gemisch enthaltend

a) mindestens eine Verbindung der Formeln I oder II gemäss Anspruch 1,

b) eine für die Härtung besagten Gemisches ausreichende Menge eines Epoxidhärters und
c) gegebenenfalls einen Härtungsbeschleuniger.

11. Härtbares Gemisch enthaltend
a) mindestens eine Verbindung der Formel X gemäss Anspruch 9,
b) eine für die Härtung besagten Gemisches ausreichende Menge eines Epoxidhärters und
c) gegebenenfalls einen Härtungsbeschleuniger.

12. Verwendung von einer oder mehreren Verbindungen der Formeln I oder II gemäss Anspruch 1 in härtbaren Epoxidharzmischungen als Anstrichmittel, Sinterpulverlacke, Pressmassen, Tauchharze, Giessharze, Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate.

13. Verwendung gemäss Anspruch 12 als Sinterpulverlacke, Imprägnier-und Laminierharze.

14. Verwendung von einer oder mehreren Verbindungen der Formel X gemäss Anspruch 9 in härtbaren Epoxidharzmischungen als Anstrichmittel, Sinterpulverlacke, Pressmassen, Tauchharze, Giessharze, Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate.

15. Verwendung gemäss Anspruch 14 als Sinterpulverlacke, Imprägnier-und Laminierharze.

16. Verwendung einer Verbindung der Formel VIII, worin $R^3$, $R^4$, $R^5$ und $R^6$ unabhängig voneinander Wasserstoff, $C_1$-$C_6$-Alkyl, Chlor oder Brom bedeuten und $R^{7'}$ ein Rest der Formel III, IV, V oder VI ist, als Härter für härtbare Epoxidharze.

Patentansprüche für den folgenden Vertragsstaat: ES

1. Verfahren zur Herstellung von Verbindungen der Formel I oder II,

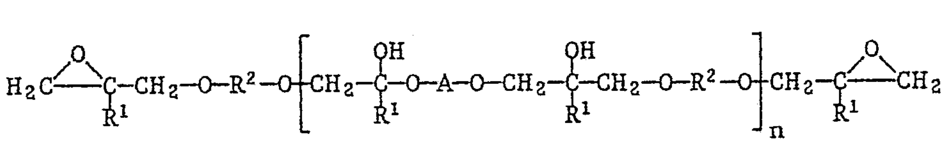 (I),

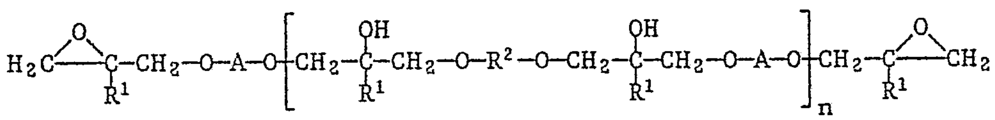 (II),

worin A ein Rest

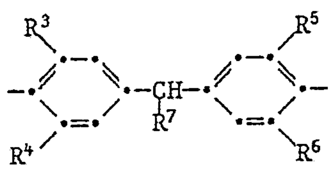

ist, $R^1$ Wasserstoff oder Methyl bedeutet,
$R^2$ der Rest eines aliphatischen, cycloaliphatischen, aromatischen oder araliphatischen Diols nach dem Entfernen beider Hydroxylgruppen ist,
$R^3$, $R^4$, $R^5$ und $R^6$ unabhängig voneinander Wasserstoff, $C_1$-$C_6$-Alkyl, Chlor oder Brom bedeuten,
$R^7$ ein Rest der Formel III, IV, V oder VI ist

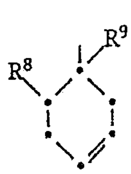 (III), 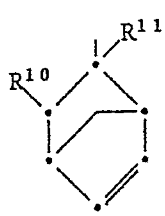 (IV), 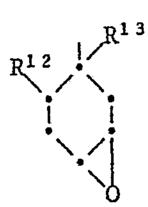 (V), 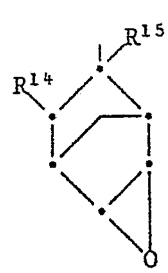 (VI),

$R^8$, $R^{10}$, $R^{12}$ und $R^{14}$ Wasserstoff, $C_1$-$C_6$-Alkyl oder Phenyl sind,
$R^9$, $R^{11}$, $R^{13}$ und $R^{15}$ Wasserstoff oder $C_1$-$C_6$-Alkyl bedeuten und der mittlere Wert von n (Zahlenmittel) eine Zahl von 1 bis 20 ist,
wobei die Reste $R^1$ bis $R^{15}$ innerhalb eines gegebenen Moleküls im Rahmen der gegebenen Definitionen unterschiedliche Bedeutungen annehmen können, dadurch gekennzeichnet, dass man n Mole eines

Diphenols der Formel VIII,

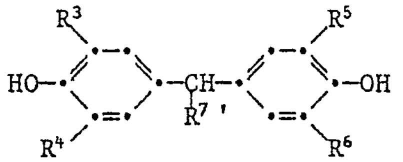

(VIII),

worin $R^3$, $R^4$, $R^5$ und $R^6$ die vorstehend angegebene Bedeutung haben und $R^{7'}$ ein Rest der Formeln III oder IV ist, mit n + 1 Molen eines Diepoxids der Formel IX,

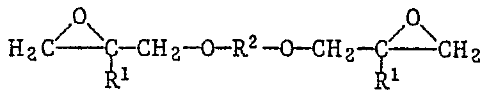

(IX),

worin $R^1$, $R^2$ und n die vorstehend angegebene Bedeutung haben, umsetzt und im Fall, dass $R^7$ in der Verbindung der Formel I ein Rest der Formeln V oder VI ist, anschliessend mit Persäure epoxidiert oder n Mole eines Diols der Formel XI,

HO-$R^2$-OH (XI),

worin n und $R^2$ die vorstehend angegebene Bedeutung haben, mit n + 1 Molen eines Diglycidylethers der Formel X,

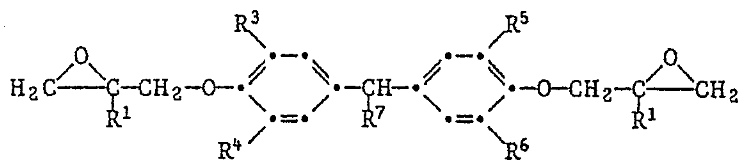

(X),

worin $R^1$, $R^2$, $R^3$, $R^4$, $R^5$, $R^6$ und $R^7$ die vorstehend angegebene Bedeutung haben, umsetzt, wobei im Fall, dass $R^7$ in der Verbindung der Formel II ein Rest der Formeln V oder VI ist, zuerst die Verbindung der Formel II mit $R^7$ als Rest der Formeln III oder IV hergestellt und anschliessend mit Persäure epoxidiert wird, oder direkt eine Verbindung der Formel X mit $R^7$ als Rest der Formeln V oder VI umgesetzt wird.

2. Verfahren gemäss Anspruch 1, worin in den Verbindungen der Fomeln I oder II $R^1$ Wasserstoff ist.

3. Verfahren gemäss Anspruch 1, worin in den Verbindungen der Formeln I oder II $R^2$ 1,2-, 1,3- oder 1,4-Phenylen ist oder Reste der Formel VII darstellt

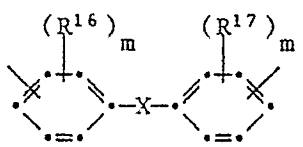

(VII),

worin X eine direkte C-C-Bindung ist oder ausgewählt wird aus der Gruppe der Reste bestehend aus $-CH_2-$, $-CHCH_3-$, $-C(CH_3)_2-$, -O-, -S-, $-SO_2-$ oder -CO-, m eine ganze Zahl von 0 bis 4 ist und $R^{16}$ und $R^{17}$ unabhängig voneinander $C_1$-$C_6$-Alkyl, Chlor oder Brom bedeuten.

4. Verfahren gemäss Anspruch 1, worin in den Verbindungen der Formeln I oder II $R^2$ einen Rest der Formel VIIa darstellt

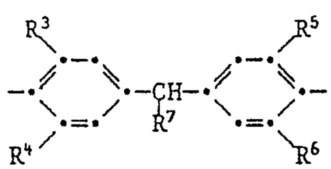

(VIIa),

und $R^3$, $R^4$, $R^5$, $R^6$ und $R^7$ die in Anspruch 1 definierte Bedeutung besitzen.

5. Verfahren gemäss Anspruch 1, worin in den Verbindungen der Formeln I oder II der Mittelwert von n eine Zahl von 1 bis 10 ist.

6. Verfahren gemäss Anspruch 1, worin in den Verbindungen der Formeln I oder II $R^7$ ein Rest der Formel III oder IV ist.

7. Verfahren gemäss Anspruch 1, worin in den Verbindungen der Formeln I oder II $R^7$ ein Rest der

Formel V oder VI ist, worin $R^{12}$, $R^{13}$, $R^{14}$ und $R^{15}$ Wasserstoff sind.

8. Verfahren zur Herstellung von Verbindungen der Formel VIII

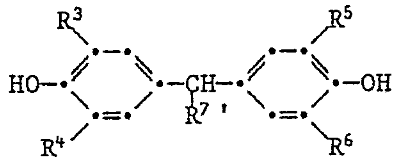

(VIII),

worin $R^3$, $R^4$, $R^5$ und $R^6$ die in Anspruch 1 definierten Bedeutungen besitzen und $R^{7'}$ ein Rest der Formel III oder IV gemäss Anspruch 1 ist durch Umsetzung von einem Aldehyd der Formel VIIIa

$R^{7'}$-CHO (VIIIa),

worin $R^{7'}$ die oben definierte Bedeutung besitzt, mit einem 4- bis 8-fachen molaren Ueberschuss, bezogen auf den Aldehyd, eines Phenols der Formel VIIIb oder eines Gemisches dieser Phenole

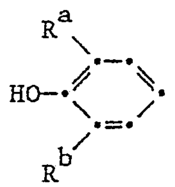

(VIIIb),

worin $R^a$ und $R^b$ die Bedeutung von $R^3$, $R^4$, $R^5$ und $R^6$ gemäss Anspruch 1 besitzen, dadurch gekennzeichnet, dass man einen einkernigen aromatischen Kohlenwasserstoff als Lösungsmittel und eine aromatische Sulfonsäure als Katalysator verwendet.

9. Verfahren zur Herstellung von Verbindungen der Formel X,

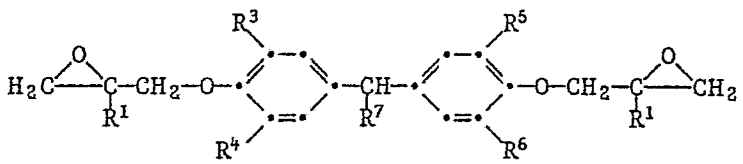

(X),

worin $R^1$, $R^3$, $R^4$, $R^5$ und $R^6$ die in Anspruch 1 definierte Bedeutung besitzen und $R^7$ ein Rest der Formeln IV oder VI gemäss Anspruch 1 ist, dadurch gekennzeichnet, dass man ein Diphenol der Formel VIII

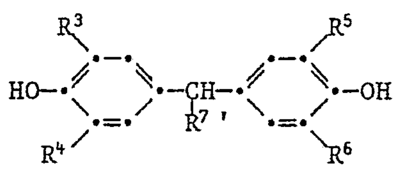

(VIII),

worin $R^3$, $R^4$, $R^5$ und $R^6$ die vorstehend angegebene Bedeutung haben und $R^7$ ein Rest der Formel IV gemäss Anspruch 1 ist, mit Epichlorhydrin oder mit β-Methylepichlorhydrin umsetzt und im Fall, dass $R^7$ in der Verbindung der Formel X ein Rest der Formel VI bedeutet, anschliessend mit Persäure epoxidiert.

10. Härtbares Gemisch enthaltend
   a) mindestens eine Verbindung der Formeln I oder II gemäss Anspruch 1,
   b) eine für die Härtung besagten Gemisches ausreichende Menge eines Epoxidhärters und
   c) gegebenenfalls einen Härtungsbeschleuniger.

11. Härtbares Gemisch enthaltend
   a) mindestens eine Verbindung der Formel X gemäss Anspruch 9,
   b) eine für die Härtung besagten Gemisches ausreichende Menge eines Epoxidhärters und
   c) gegebenenfalls einen Härtungsbeschleuniger.

12. Verwendung von einer oder mehreren Verbindungen der Formeln I oder II gemäss Anspruch 1 in härtbaren Epoxidharzmischungen als Anstrichmittel, Sinterpulverlacke, Pressmassen, Tauchharze, Giessharze, Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate.

13. Verwendung gemäss Anspruch 12 als Sinterpulverlacke, Imprägnier-und Laminierharze.

14. Verwendung von einer oder mehreren Verbindungen der Formel X gemäss Anspruch 9 in härtbaren Epoxidharzmischungen als Anstrichmittel, Sinterpulverlacke, Pressmassen, Tauchharze, Giessharze,

Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate.

15. Verwendung gemäss Anspruch 14 als Sinterpulverlacke, Imprägnier-und Laminierharze.

16. Verwendung einer Verbindung der Formel VIII, worin $R^3$, $R^4$, $R^5$ und $R^6$ unabhängig voneinander Wasserstoff, $C_1$-$C_6$-Alkyl, Chlor oder Brom bedeuten und $R^{7'}$ ein Rest der Formel III, IV, V oder VI ist, als Härter für härtbare Epoxidharze.